Europäisches Patentamt

(19) European Patent Office

Office européen des brevets

(11) Veröffentlichungsnummer : **0 527 163 B1**

(12) # EUROPÄISCHE PATENTSCHRIFT

(45) Veröffentlichungstag der Patentschrift :
**12.01.94 Patentblatt 94/02**

(51) Int. Cl.$^5$ : **A61C 1/00**

(21) Anmeldenummer : **91908262.8**

(22) Anmeldetag : **03.05.91**

(86) Internationale Anmeldenummer :
**PCT/EP91/00831**

(87) Internationale Veröffentlichungsnummer :
**WO 91/16863 14.11.91 Gazette 91/26**

(54) **VORRICHTUNG ZUM ENTFERNEN VON KARIÖSEM ZAHNMATERIAL MIT LASERLICHT.**

(30) Priorität : **04.05.90 DE 4014303**

(43) Veröffentlichungstag der Anmeldung :
**17.02.93 Patentblatt 93/07**

(45) Bekanntmachung des Hinweises auf die
Patenterteilung :
**12.01.94 Patentblatt 94/02**

(84) Benannte Vertragsstaaten :
**CH DE FR GB IT LI**

(56) Entgegenhaltungen :
**EP-A- 0 375 578**
**WO-A-89/01317**
**WO-A-90/00035**

(56) Entgegenhaltungen :
**WO-A-90/01907**
**DE-A- 4 030 734**
**FR-A- 2 598 608**
**FR-B- 1 392 614**

(73) Patentinhaber : **RECHMANN, Peter**
**Dellestrasse 79**
**D-40627 Düsseldorf (DE)**

(72) Erfinder : **RECHMANN, Peter**
**Dellestrasse 79**
**D-40627 Düsseldorf (DE)**

(74) Vertreter : **Dallmeyer, Georg et al**
**Patentanwälte Von Kreisler-Selting-Werner**
**Postfach 10 22 41**
**D-50462 Köln (DE)**

## Beschreibung

Die Erfindung geht aus von einer Vorrichtung zum Entfernen von kariösem Zahnmaterial mit Laserlicht nach dem Oberbegriff des Anspruchs 1 sowie der Verwendung einer Laserlichtquelle nach dem Oberbegriff des Anspruchs 14.

Aus der DE-A-38 00 555 ist die Verwendung eines ultravioletten Laserlichts mit einer Wellenlänge von 193 nm bekannt, das von einem Argonfluorid-Excimer-Laser emittiert wird. Die Photonenenergie des Laserlichts wird auf Größen kumuliert, die größer sind als die Bindungsenergien der jeweils aufzulösenden Zahnhartsubstanzen. Bei einer Energiedichte von mindestens 5000 $mJ/mm^2$ pro Puls unter Fokussierung auf einen Lichtfleck von ca. 1 - 2 $mm^2$ soll es möglich sein, eine Impulsenergiedichte zu erzeugen, bei welcher der Schwellenwert zum Eintritt der Photodekomposition in ausreichender Größenordnung überschritten wird. Für die Repetitionsrate der Laserimpulse wird vorgeschlagen, eine Frequenz von kleiner als 100 Hz zu wählen. Die Laserimpulsdauer beträgt ca. 15 bis 20 ns. Das bekannte Verfahren soll die kontrollierte Entfernung von gesunden oder kariösen Zahnhartsubstanzen ermöglichen.

Aus der US-A-4 521 194 und der US-A-4 818 230 ist es bekannt, einen Yttrium-Aluminium-Granat-Laser (Wellenlänge 1064 nm) zu verwenden, dessen Ausgangsenergie im Bereich zwischen 1 und 100 mJ bei einer Impulsdauer von einigen Picosekunden bis zu einigen Millisekunden und dessen Lichtstrahldurchmesser im Bereich zwischen 50 und 2000 µm liegt.

Die bekannten Vorrichtungen ermöglichen es, sowohl kariöses Dentin als auch nicht kariös erweichtes Dentin zu bearbeiten. Der Nachteil dabei ist, daß, wie beim herkömmlichen Bohren, bei der Kariesentfernung auch gesunde Zahnhartsubstanz entfernt wird, so daß ein unnötiger Abbau der Zahnhartsubstanz erfolgt.

Ein weiterer Nachteil gemäß dem bekannten Stand der Technik besteht darin, daß eine hohe Energiedichte eingebracht wird, so daß die thermische Belastung des Zahnes auch zu einer Schädigung benachbarter Bereiche führen kann.

Ein weiteres Problem entsteht bei Laservorrichtungen, die eine Fokussierung des Laserstrahls erfordern: Bei diesen Vorrichtungen ist die exakte Einhaltung eines definierten Abstandes erforderlich, um den Lichtfleckdurchmesser und damit die eingebrachte Energiedichte konstant zu halten. Hierzu sind aufwendige und die zahnärztliche Arbeit behindernde Vorrichtungen nötig.

Aus der nicht vorveröffentlichten EP-A-0 375 578 ist die Verwendung eines Nd:YAG-Lasers mit einer Wellenlänge von 532 nm zur Zahnbehandlung bekannt. Die Ausgangsenergie des Lasers beträgt pro Impuls 10 bis 50 mJ bei einer Frequenz von 50 Hz. Die Impulsdauer beträgt zwischen 100 und 300 µs. Der Laserstrahl wird über einen konvergierenden Spiegel auf einen Lichtfleckdurchmesser zwischen 0,2 und 0,6 mm fokussiert.

Der Erfindung liegt die Aufgabe zugrunde, eine Vorrichtung zum Entfernen von kariösem Zahnmaterial zu schaffen, mit der bei geringstmöglicher Energie eine selektive vollständige Entfernung von Dentinkaries bei größtmöglicher Schonung der gesunden Zahnhartsubstanz ermöglicht wird, sowie die Verwendung spezieller Laserparameter hierfür aufzuzeigen.

Zur Lösung dieser Aufgabe ist bei einer Vorrichtung zum Entfernen von kariösem Zahnmaterial mit einem von einer gepulsten Laserlichtquelle erzeugten Laserlichtstrahl und einer flexiblen Laserlichtleitung, über die der Laserlichtstrahl einen Lichtfleck auf einem zu behandelnden Zahn erzeugt, erfindungsgemäß vorgesehen, daß die Laserlichtquelle Laserlicht mit einer Wellenlänge im Bereich zwischen 320 und 520 nm emittiert und daß das gepulste Laserlicht pro Laserlichtimpuls eine Energiedichte im Lichtfleck von ca. 0,14 bis ca. 7,0 $J/cm^2$ aufweist.

Die Verwendung einer Wellenlänge im Bereich zwischen 320 und 520 nm ermöglicht ein selektives Abtragen von kariöser Substanz, da in diesem Spektralbereich Dentinkaries das Laserlicht stärker absorbiert als gesundes Dentin.

Die Ablationsschwellen für Dentinkaries und gesundes Dentin weisen in diesem Bereich den größten Unterschied auf, so daß die relativ weichere Dentinkaries infolge einer reduzierbaren Energiedichte bei geringster Wärmebelastung selektiv entfernt werden kann.

Vorzugsweise ist die Laserlichtleitung im Kontaktmode oder im Abstand von einigen Millimetern auf den zu behandelnden Zahn richtbar. Auf Grund der nicht erforderlichen Fokussierung kann der Laserstrahl im Kontaktmode auf die zu bearbeitende Stelle gesetzt werden, was ein präzises Arbeiten ermöglicht und aufwendige Abstandhalter erübrigt. Außerdem kann auch mit Abstand von der Ablationsstelle gearbeitet werden. Ein wesentlicher Vorteil ist dabei, daß trotz Abstandsänderungen von einigen Millimetern noch Energiedichten oberhalb der Ablationsschwelle von kariösem Dentin erreicht werden.

Vorzugsweise wird eine Energiedichte pro Laserlichtimpuls im Bereich zwischen 0,175 und 2,0 $J/cm^2$ verwendet. Eine bevorzugte Wellenlänge beträgt ca. 355 nm bis 375 nm. Im Bereich dieser Wellenlänge besteht ein Maximum im Absorptionsunterschied zwischen gesundem Dentin und Dentinkaries, so daß mit geringerer Energiedichte ein Optimum an Selektion zwischen Dentin und Dentinkaries erreicht werden kann. Weicht die Wellenlänge des Laserlichtes von der optimalen Wellenlänge ab, kann ein von der Wellenlänge λ abhängiger Energiedichtekorrekturfaktor K verwendet werden. Bei dieser korrigierten Energiedichte ergibt sich auch bei vom Optimum abweichenden Wellenlängen noch eine zufriedenstellende Selektion, bei der nur kariöses Dentin und praktisch kein gesundes Dentin abgetragen wird.

Die Verwendung eines Lasers zum Entfernen von Zahnhartsubstanz, Dentin und Dentinkaries ist erfindungsgemäß gekennzeichnet durch die selektive Ablation von Dentinkaries unter Verwendung einer Wellenlänge von ca. 350 - 410 nm, vorzugsweise ca. 375 nm und einer Energiedichte im Lichtfleck pro Laserlichtimpuls im Bereich zwischen 0,35 und 1,7 J/cm².

Bei einer Impulsdauer von mehr als 50 ns wird die Energie vorzugsweise um einen Faktor F erhöht, der proportional zur Wurzel aus dem Quotienten der eingestellten Impulsdauer und einer Basis-Impulsdauer von 10 ns ist. Dies bedeutet, daß bei einer Impulsdauer I folgende Beziehung für F gilt:

$$F \sim \sqrt{\frac{I[s]}{10^{-8}[s]}}$$

Der Faktor berücksichtigt den ab ca. 50 ns einsetzenden Effekt der thermischen Diffusion.

Bei einem Ausführungsbeispiel ist die Verwendung einer Repetitionsfrequenz unter 12 Hz vorgesehen. Bei Frequenzen unter 12 Hz kann auf eine zusätzliche Kühlung verzichtet werden.

Bei einem anderen Ausführungsbeispiel wird eine Repetitionsfrequenz zwischen 100 und 200 Hz bevorzugt.

Vorzugsweise wird ein frequenzverdoppelter Alexandrit-Laser verwendet.

Im folgenden wird unter Bezugnahme auf die Zeichnungen ein Ausführungsbeispiel der Erfindung näher erläutert. Es zeigen:

Fig. 1    die Transmissionsspektren von kariösem und gesundem, nicht kariös erweichten Dentin sowie des Zahnschmelzes,

Fig. 2    den Absorptionsfaktor Dentinkaries zu Dentin in Abhängigkeit von der Wellenlänge des Laserlichts, und

Fig. 3    den Ablationsdruck von Dentin und Dentinkaries in Abhängigkeit von der Energiedichte.

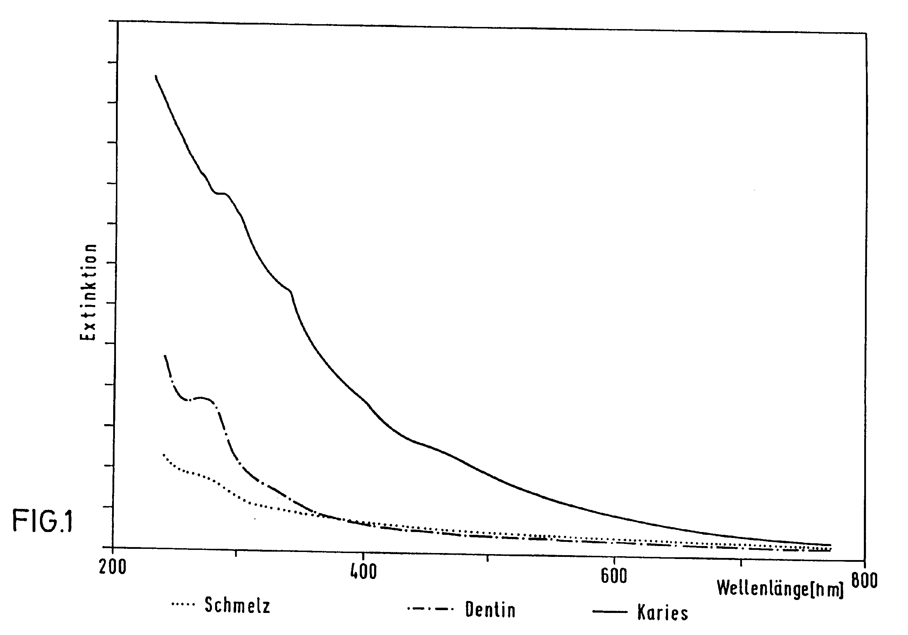

In Fig. 1 sind die Transmissionsspektren des kariösen Dentins (ausgezogene Linie), des gesunden nicht kariös erweichten Dentins (strichpunktierte Linie) und des Zahnschmelzes (gepunktete Linie) dargestellt.

Auf der Ordinate ist die Extinktion und auf der Abszisse die Wellenlänge des Lichtes aufgetragen.

Die Transmissionsspektren zeigen, daß im dargestellten Wellenlängenbereich zwischen 240 und 770 nm das kariöse Dentin das Laserlicht deutlich stärker absorbiert als das nicht kariös erweichte Dentin. In beiden Transmissionsspektren finden sich im Wellenlängenbereich zwischen 250 und 320 nm die charakteristischen UV-Absorptionseigenschaften von aromatischen Amino- und Nukleinsäuren mit maximalen Absorptionen um 280 nm. Zahnschmelz besteht zu 96 - 98 % aus anorganischen Komponenten und besitzt folglich keine ausgeprägte Absorptionsbande.

Im längerwelligen Bereich über 320 nm zeigen Proteine keine wahre Absorption, wenn sie nicht mit einem Koenzym oder einer prosthetischen Gruppe in Konjugation stehen.

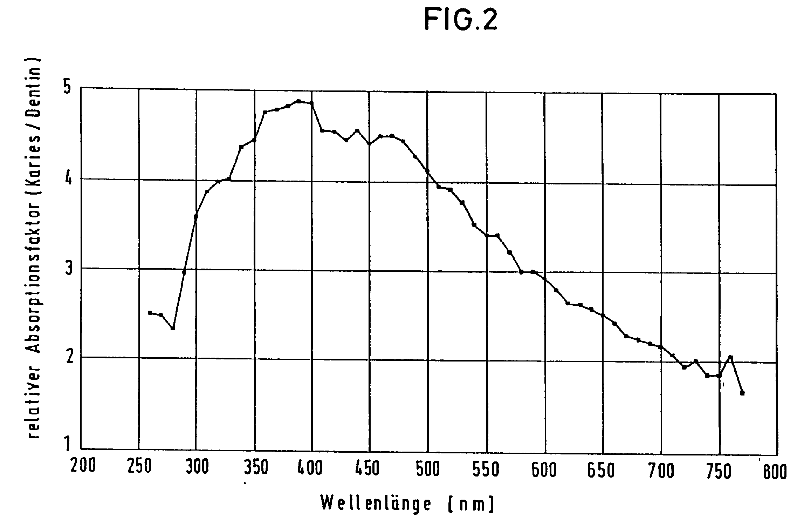

In Fig. 2 ist auf der Abszisse die Wellenlänge des eingestrahlten Lichtes aufgetragen. Die Ordinate stellt die optische Dichte von Karies im Verhältnis zur optischen Dichte von Dentin dar. Der Wert 1 bedeutet hierbei, daß kein Unterschied zwischen der optischen Dichte von Karies und nicht kariös erweichtem Dentin besteht.

Im Spektralbereich von 320 bis 520 nm finden sich die größten relativen Absorptionsunterschiede. Kariös erweichtes Dentin absorbiert 4-5mal stärker als nicht kariös erweichtes Dentin.

Die Verwendung einer Wellenlänge im Bereich zwischen 320 und 520 nm, ermöglicht ein selektives Abtragen von kariöser Substanz.

Zur selektiven Abtragung kariös erweichten Dentins muß die eingestrahlte Energiedichte unterhalb der Ablationsschwelle von nicht kariös verändertem Dentin liegen.

Um Karies abzutragen, muß in Abhängigkeit von der benutzten Wellenlänge die Energiedichte des Laserpulses variiert werden. Mit steigender Wellenlänge des Laserlichtes steigen die Ablationsschwellen von Dentin und Karies in etwa gleichermaßen. Das Verhältnis zwischen den Schwellenwerten für eine Ablation von Dentinkaries bzw. Dentin bleibt aber über den bevorzugten Wellenlängenbereichen zwischen 320 und 520 nm im wesentlichen gleich.

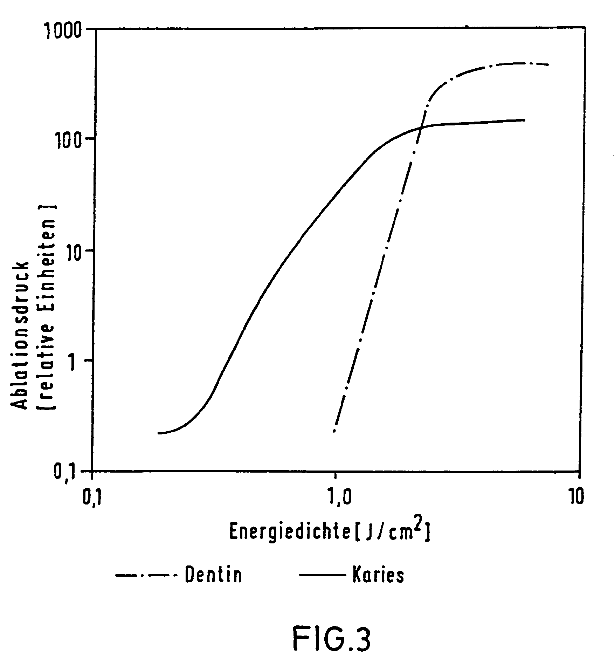

Als Lichtquelle wurde zur Ermittlung des Zusammenhangs gemäß Fig. 3 ein frequenzverdreifachter und gütegeschalteter Nd:YAG-Laser verwendet, der Laserlicht mit einer Wellenlänge von 355 nm und einer Lichtimpulslänge von 9 ns emittiert.

Fig. 3 zeigt die Abhängigkeit des Ablationsdruckes von der Energiedichte des Laserlichtes von kariösem Dentin (ausgezogene Linie) und nicht kariös erweichtem Dentin (strichpunktierte Linie) bei einer Impulsdauer

3

von 9 ns für den im Ausführungsbeispiel verwendeten Laser. Die Kurve für nicht kariös erweichtes Dentin ist zu höheren Energiedichten hin verschoben. Der Energie-Schwellenwert für die Ablation von Dentinkaries liegt bei einer Impulsdauer von 9 ns bei einem Wert von ca. 0,35 J/cm². Dagegen liegt der Schwellenwert für gesundes Dentin oberhalb einer Energiedichte von 1,0 J/cm². Hier ergibt sich somit ein Verhältnis von ca. 3 : 1 für die Ablationsschwellen von Dentinkaries und nicht kariös erweichtem Dentin.

Arbeitet man bei einer Wellenlänge von ca. 355 nm bis 375 nm mit Energiedichten zwischen ca. 0,35 und 1,0 J/cm², so wird selektiv Dentinkaries abgetragen. Das nicht kariöse Dentin wird geschont (Fig. 3).

Die Energiedichte beträgt bei einem bevorzugten Ausführungsbeispiel mit einer Wellenlänge von 375 nm 1,3 J/cm².

Für den Wellenlängenbereich von 355 nm wurde eine optische Dichte von ca. 1,0 (Tabelle) ermittelt. Soll nun Laserlicht z.B. mit einer Wellenlänge von 520 nm benutzt werden, so kann man aus der Tabelle den Wert für die optische Dichte von ca. 0,3 entnehmen.

Dies bedeutet, daß ungefähr nur ein Drittel der eingestrahlten Energie im Vergleich zur Wellenlänge 355 nm absorbiert wird. Die Ablationsschwellen verschieben sich somit um den Faktor 3 zu höheren Werten. Die Selektivität für die Abtragung von kariös erweichtem Dentin bleibt jedoch erhalten. Die Energiedichten für selektive Kariesabtragung liegen bei dieser Wellenlänge (520 nm) zwischen ca. 1,2 und 3,3 J/cm².

Der Tabelle kann für jede Wellenlänge im bevorzugten Bereich von 320 bis 520 nm ein Faktor entnommen werden, mit dessen Hilfe die benötigte Energiedichte zur selektiven Ablation von Dentinkaries berechnet werden kann. Multipliziert man den Energiedichtenbereich, in dem selektive Ablation bei 355 nm erfolgt (0,35 bis 1,0 J/cm²), mit dem entsprechenden Faktor K der gewünschten Wellenlänge, so erhält man die notwendigen Energiedichten für eine selektive Kariesentfernung bei dieser Wellenlänge.

Im Bereich zwischen 330 und 480 nm läßt sich der Energiedichtekorrekturfaktor K aus

$$K = 0,0115 \ nm^{1-} \cdot \lambda - 3$$

berechnen, wobei Abweichungen von ±10 % des Korrekturfaktors auf Grund von Meßungenauigkeiten möglich sind.

## Tabelle

| Wellenlänge in nm | Energiedichtenkorrekturfaktor K |
|---|---|
| 320 | 0,8 |
| 330 | 0,8 |
| 340 | 0,9 |
| 350 | 1,0 |
| 360 | 1,1 |
| 370 | 1,2 |
| 380 | 1,3 |
| 390 | 1,4 |
| 400 | 1,5 |
| 410 | 1,7 |
| 420 | 1,8 |
| 430 | 1,9 |
| 440 | 2,0 |
| 450 | 2,1 |
| 460 | 2,2 |
| 470 | 2,4 |
| 480 | 2,5 |
| 490 | 2,7 |
| 500 | 2,9 |
| 510 | 3,1 |
| 520 | 3,3 |

Die Meßgenauigkeit der Absolutwerte für die Energiedichten ist durch die Ungenauigkeiten der meßtechnischen Möglichkeiten begrenzt. Die Begrenzungen liegen in der Meßgenauigkeit des Energiemeters, der Bestimmung der bestrahlten Fläche sowie in Inhomogenitäten innerhalb des Strahlprofils des Lasers.

Bei Bedarf ist die Impulslänge des Laserlichtes variabel. Sie sollte jedoch 10 μs nicht überschreiten, da sonst eine thermische Schädigung des angrenzenden Gewebes nicht ausgeschlossen werden kann.

Bei einer Impulslänge des Laserlichtes über 50 ns ist ein Erhöhungsfaktor F für die Energiedichte vorzugsweise anzuwenden, der proportional zur Wurzel aus dem Quotienten der eingestellten Impulslänge und einer Basis-Impulslänge von 10 ns ist. Der Proportionalfaktor ist vorzugsweise 1.

Die zur selektiven Ablation von Dentinkaries benötigten Lichtenergiedichten können durch Lichtleiter, deren Durchmesser der Ausdehnung der kariösen Läsion angepaßt werden können, geleitet werden; die Einbeziehung der Lichtleiter in zahnärztliche Winkelstücke ist möglich. Die Konstruktion von aufwendigen Applikationsinstrumenten ist daher nicht erforderlich. Die Ablation von Karies kann sowohl im Kontaktmode als auch im Abstand von einigen Millimetern ausgeführt werden.

Es empfiehlt sich, kolinear mit dem unsichtbaren Laserlichtstrahl einen sichtbaren Pilotstrahl vorzusehen.

**Patentansprüche**

1. Vorrichtung zum Entfernen von kariösem Zahnmaterial mit einer gepulsten Laserlichtquelle, deren Laserlichtstrahl über eine flexible Laserlichtleitung einen Lichtfleck auf einem zu behandelnden Zahn erzeugt, wobei das gepulste Laserlicht pro Laserlichtimpuls eine Energiedichte im Lichtfleck von 0,14 bis 7,0 J/cm² aufweist
   **dadurch gekennzeichnet,**
   daß die Laserlichtquelle Laserlicht mit einer Wellenlänge im Bereich zwischen 320 und 520 nm emittiert.

2. Vorrichtung nach Anspruch 1, dadurch gekennzeichnet, daß die Energiedichte im Wellenlängenbereich zwischen 330 nm und 480 nm in Abhängigkeit von der Wellenlänge $\lambda$ um den Korrekturfaktor $K = 0{,}0115$ nm$^{-1} \cdot \lambda - 3$ korrigiert ist.

3. Vorrichtung nach einem der Ansprüche 1 oder 2, dadurch gekennzeichnet, daß die Impulsdauer des Laserlichts weniger als 10 µs beträgt.

4. Vorrichtung nach einem der Ansprüche 1 bis 3, dadurch gekennzeichnet, daß die Impulsdauer des Laserlichts zwischen 50 ns und 5 µs beträgt.

5. Vorrichtung nach einem der Ansprüche 1 bis 3, dadurch gekennzeichnet, daß die Impulsdauer des Laserlichts 9 ns beträgt.

6. Vorrichtung nach einem der Ansprüche 1 bis 4, dadurch gekennzeichnet, daß bei einer Impulsdauerlänge von mehr als 50 ns die Energiedichte um einen Faktor F erhöht wird, der der Wurzel aus dem Quotienten der eingestellten Impulsdauer und einer Impulsdauer von 10 ns proportional ist.

7. Vorrichtung nach einem der Ansprüche 1 bis 6, dadurch gekennzeichnet, daß die Repetitionsfrequenz der Laserlichtimpulse maximal 12 Hz beträgt.

8. Vorrichtung nach den Ansprüchen 1 bis 6, dadurch gekennzeichnet, daß die Repetitionsfrequenz der Laserlichtimpulse 100 bis 200 Hz beträgt.

9. Vorrichtung nach einem der Ansprüche 1 bis 8, dadurch gekennzeichnet, daß die Laserlichtleitung im Kontaktmode oder im Abstand von einigen Millimetern auf den zu behandelnden Zahn richtbar ist.

10. Vorrichtung nach einem der Ansprüche 1 bis 9, dadurch gekennzeichnet, daß das gepulste Laserlicht pro Laserlichtimpuls eine Energiedichte im Lichtfleck von 0,175 bis 2,0 J/cm² aufweist.

11. Vorrichtung nach einem der Ansprüche 1 bis 10, dadurch gekennzeichnet, daß die Laserlichtquelle Laserlicht mit einer Wellenlänge im Bereich zwischen 350 nm bis 410 mm mit einer Energiedichte pro Laserlichtimpuls im Lichtfleck im Bereich zwischen 0,35 und 1,7 J/cm² emittiert.

12. Vorrichtung nach einem der Ansprüche 1 bis 11, dadurch gekennzeichnet, daß die Wellenlänge des Laserlichts 355 nm bis 375 nm beträgt.

13. Vorrichtung nach einem der Ansprüche 1 bis 12, dadurch gekennzeichnet, daß das gepulste Laserlicht pro Laserlichtimpuls eine Energiedichte im Lichtfleck von 0,35 bis ca. 1,0 J/cm² aufweist.

14. Vorrichtung nach einem der Ansprüche 1 bis 12, dadurch gekennzeichnet, daß bei einer Wellenlänge von 375 nm die Energiedichte 1,3 J/cm² beträgt.

15. Vorrichtung nach einem der Ansprüche 1 bis 14, gekennzeichnet, daß die Laserlichtquelle ein frequenzverdreifachter Nd:YAG-Laser ist.

16. Vorrichtung nach einem der Ansprüche 1 bis 14, gekennzeichnet, daß die Laserlichtquelle ein frequenzverdoppelter Alexandrit-Laser ist.

**Claims**

1. A device for removing carious tooth material with a pulsed laser light source, the laser light beam of which, via a flexible laser light guide, produces a light spot on a tooth to be treated, said pulsed laser light having, per laser light pulse, an energy density in said light spot of about 0.14 to about 7.0 J/cm$^2$, characterised in
that said laser light source emits laser light with a wavelength in the range from 320 to 520 nm.

2. The device of claim 1, characterised in that the energy density in the wavelength range between 330 nm and 480 nm is corrected in dependence on the wavelength $\lambda$ by the correction factor
$$K = 0.115 \, \text{nm}^{-1} \cdot \lambda - 3.$$

3. The device of one of claims 1 or 2, characterised in that the pulse duration of the laser light is less than 10 $\mu$s.

4. The device of one of claims 1 to 3, characterised in that the pulse duration of the laser light is between 50 ns and 5 $\mu$s.

5. The device of one of claims 1 to 3, characterised in that the pulse duration of the laser light is 9 ns.

6. The device of one of claims 1 to 4, characterised in that, at a pulse duration of more than 50 ns, the energy density is increased by a factor F that is proportional to the root of the ratio of the set pulse duration and a pulse duration of 10 ns.

7. The device of one of claims 1 to 6, characterised in that the repetition frequency of said laser light pulses is 12 Hz at most.

8. The device of one of claims 1 to 6, characterised in that the repetition frequency of said laser light pulses is 100 to 200 Hz.

9. The device of one of claims 1 to 8, characterised in that said laser light guide may be directed onto the tooth to be treated either in a contact mode or at a distance of several millimeters.

10. The device of one of claims 1 to 9, characterised in that, per pulse, said pulsed laser light has an energy density in said light spot of 0.175 to 2.0 J/cm$^2$.

11. The device of one of claims 1 to 10, characterised in that said laser light source emits laser light with a wavelength in the range between 350 nm and 410 nm with an energy density per laser light pulse in the light spot in the range between 0.35 and 1.7 J/cm$^2$.

12. The device of one of claims 1 to 11, characterised in that the wavelength of the laser light is 355 nm to 375 nm.

13. The device of one of claims 1 to 12, characterised in that, per pulse, said pulsed laser light has an energy density in said light spot of 0.35 to about 1.0 J/cm$^2$.

14. The device of one of claims 1 to 12, characterised in that, at a wavelength of 375 nm, the energy density is 1.3 J/cm$^2$.

15. The device of one of claims 1 to 14, characterised in that the laser light source is a frequency-tripled Nd:YAG laser.

16. The device of one of claims 1 to 14, characterised in that the laser light source is a frequency-doubled alexandrite laser.

**Revendications**

1. Dispositif pour l'élimination d'un matière dentaire cariée à l'aide d'une source de lumière laser pulsée dont le rayonnement laser produit, par l'intermédiaire d'un conduit de lumière laser flexible, un point lumineux sur une dent à traiter, la lumière laser pulsée présentant, par impulsion de lumière laser, une densité d'énergie au point lumineux de 0,14 à 7,0 J/cm$^2$, caractérisé en ce que la source de lumière laser émet de la lumière laser avec une longueur d'onde dans la plage entre 320 et 520 nm.

2.  Dispositif suivant la revendication 1, caractérisé en ce que la densité d'énergie dans la plage de longueurs d'onde entre 330 nm et 480 nm est corrigée, en fonction de la longueur d'onde λ, du facteur de correction K = 0,0115 nm$^{-1}$ · λ- 3.

3.  Dispositif suivant l'une des revendications 1 ou 2, caractérisé en ce que la durée d'impulsion de la lumière laser est inférieure à 10 μs.

4.  Dispositif suivant l'une des revendications 1 à 3, caractérisé en ce que la durée d'impulsion de la lumière laser est comprise entre 50 ns et 5 μs.

5.  Dispositif suivant l'une des revendications 1 à 3, caractérisé en ce que la durée d'impulsion de la lumière laser est de 9 ns.

6.  Dispositif suivant l'une des revendications 1 à 4, caractérisé en ce que pour une durée d'impulsion de plus de 50 ns, la densité d'énergie est augmentée d'un facteur F qui est proportionnel à la racine du quotient de la durée d'impulsion réglée et d'une durée d'impulsion de 10 ns.

7.  Dispositif suivant l'une des revendications 1 à 6, caractérisé en ce que la fréquence maximale de répétition des impulsions de lumière laser est de 12 Hz.

8.  Dispositif suivant l'une des revendications 1 à 6, caractérisé en ce que la fréquence de répétition des impulsions de lumière laser est de 100 à 200 Hz.

9.  Dispositif suivant l'une des revendications 1 à 8, caractérisé en ce que le conduit de lumière laser peut être orienté sur la dent à traiter, en mode de contact ou à une distance de quelques millimètres.

10. Dispositif suivant l'une des revendications 1 à 9, caractérisé en ce que la lumière laser pulsée présente, par impulsion de lumière laser, une densité d'énergie au point lumineux de 0,175 à 2,0 J/cm$^2$.

11. Dispositif suivant l'une des revendications 1 à 10, caractérisé en ce que la source de lumière laser émet de la lumière laser à une longueur d'onde de l'ordre de 350 nm à 410 nm, avec une densité d'énergie par impulsion de lumière laser au point lumineux de l'ordre de 0,35 à 1,7 J/cm$^2$.

12. Dispositif suivant l'une des revendications 1 à 11, caractérisé en ce que la longueur d'onde de la lumière laser est de 355 nm à 375 nm.

13. Dispositif suivant l'une des revendications 1 à 12, caractérisé en ce que la lumière laser pulsée présente, par impulsion de lumière laser, une densité d'énergie au point lumineux de 0,35 à environ 1,0 J/cm$^2$.

14. Dispositif suivant l'une des revendications 1 à 12, caractérisé en ce qu'à une longueur d'onde de 375 nm, la densité d'énergie est de 1,3 J/cm$^2$.

15. Dispositif suivant l'une des revendications 1 à 14, caractérisé en ce que la source de lumière laser est un laser Nd:YAG à fréquence triplée.

16. Dispositif suivant l'une des revendications 1 à 14, caractérisé en ce que la source de lumière laser est un laser à alexandrite à fréquence doublée.

FIG.1

EP 0 527 163 B1

# FIG.2

FIG.3